# EUROPÄISCHE PATENTSCHRIFT

(11) **EP 2 497 513 B1**
(45) Veröffentlichungstag und Bekanntmachung des Hinweises auf die Patenterteilung: **04.11.2015**
(21) Anmeldenummer: 12167019.4
(22) Anmeldetag: 16.09.2002
(51) Int. Cl.: A61M 15/00

(54) **Inhalator für pulverförmige, insbesondere medizinische Substanzen**
Inhalator for powdery, in particular medical, substances
Inhalateur de substances en poudre, en particulier de substances médicales

(43) Veröffentlichungstag der Anmeldung: 12.09.2012
(62) Teilanmeldung aus: 02807961.4
(73) Patentinhaber: Sanofi SA, 1214 Vernier (CH)
(72) Erfinder: von Schuckmann, Alfred, 47627 Kevelaer (DE)
(74) Vertreter: Epping - Hermann - Fischer

(56) Entgegenhaltungen:
- EP-A1- 0 308 100
- EP-A1- 0 387 222
- WO-A2-01/21238
- WO-A2-97/30743
- WO-A2-98/41256
- US-A- 5 239 992
- US-A- 5 429 122

## Beschreibung

Die Erfindung bezieht sich auf einen Inhalator für pulverförmige, insbesondere medizinische Substanzen, mit einem zu einem Mundstück führenden Saugluftkanal, ferner einer Vorratskammer für die Substanz und einer linear bewegten Dosierkammer zum Abteilen einer bestimmten Substanzmenge aus der Vorratskammer in den Bereich einer Übergabestelle an den Saugluftstrom.

Ein Inhalator dieser Art ist durch die DE-OS 40 27 391 bekannt. Die auszugebende Substanzmenge wird in einer nach unten austrichternden Vorratskammer an die herausbewegbare Dosierkammer übergeben. Austräger ist ein diese aufweisender, linear beweglicher Schieber. In den Kanal des Mundstückes gestellt, dockt ein rückwärtiger Raum der Dosierkammer an ein in einer Kolben/Zylinder-Einheit gespeichertes Luftvolumen an. Unter Ausübung der Einatmung im Sinne eines Saugluftstromes wird das Luftvolumen schlagartig ausgelöst und freigegeben. Der so mechanisch strömungsunterstützte Saughub räumt die Dosierkammer entleerend frei. Der durch den Unterdruck auszulösende Druckluftausstoß erfordert einen erheblichen Bauaufwand; auch ist der plötzlich auftretende Luftstoß für die meisten Patienten zumindest sehr gewöhnungsbedürftig.

In der Druckschrift US 5,429,122 ist ein Inhalator mit einem Transportmechanismus für den Transport einer Dosis aus einem Reservoir beschrieben, bei dem der Transportmechanismus einen federbelasteten Kolben aufweist.

Aufgabe der Erfindung ist es, einen gattungsgemäßen Inhalator anzugeben, der einfach bedienbar ist.

Diese Aufgabe ist zunächst und im Wesentlichen bei einem Inhalator mit den Merkmalen des Anspruchs 1 gelöst.

Zufolge solcher Ausgestaltung ist ein baulich einfacher, funktionssicherer Inhalator erzielt. Die Einatmung erbringt einen so ausreichenden Austragsunterdruck, dass die Dosierkammer einwandfrei entleert wird. Der Darbietungsbereich an die Strömungskomponente liegt äußerst austragswirksam. Die linear bewegte Dosierkammer arbeitet schöpfend und liegt mit Erreichen der Übergabestelle so vom Vorratsbereich der pulverförmigen Substanz abgetrennt, dass nichts mehr zurückfallen kann. Der Stand der Technik versucht, diese möglicherweise zu ungleichen Chargen führende Quelle durch den Einsatz eines Siebes zum rückwärtigen Raum hin, also zum zugehenden Fremdluftstrom hin, zu verhindern.

Weitere Gegenstände sind nachstehend in Bezug zu dem Gegenstand des Anspruchs 1 erläutert. So wird weiter vorgeschlagen, dass die Dosierkammer als Querbohrung eines Verschlusskappen-abhängig verlagerbaren Dornes gestaltet ist. Die Ausgabe-Bereitschaftsstellung wird so gleichsam automatisch bewerkstelligt, einfach in Ausübung der gewohnten Handhabung des Schließens und Öffnens. Die bevorzugt durchgehende Querbohrung ist von zwei Seiten her ausräumbar. Eine besonders wirksame Maßnahme ergibt sich durch eine konische Querbohrung. Die abgeteilte Substanzmenge wird vom weitenden Ende her sogar rascher via Komponente an den Saugluftstrom übergeben. Zur Erlangung einer in die Erstreckungsrichtung der Dosierkammer leitenden Luftführung der Komponente ist es von Bedeutung, dass der Dosierkammer ein an den Saugluftstrom anschließender Luftdurchlass zugeordnet ist. Das ergibt gleichsam eine lokalisierte Zone des Unterdrucks. Günstig ist, beiden offenen Enden der Dosierkammer je ein Luftdurchlass vorzulagern. Im Falle der konischen Querbohrung wird zweckmäßig weiter so vorgegangen, dass dem Ende größeren lichten Durchmessers der Dosierkammer ein Luftdurchlass kleineren Durchmessers als dieser zugeordnet ist und dem Ende kleineren lichten Durchmessers ein Luftdurchlass größeren Durchmessers als dieser. Von der weitenden Seite her wird so zufolge größeren Unterdrucks vorrangig ausgeräumt, also gerade in der Richtung, in der durch die sich entsprechend weitende Wandung der Dosierkammer kein Reibungshindernis vorliegt. Sodann bringt die Erfindung in Vorschlag, dass die Luftdurchlässe an einem topfförmigen, den Dorn führenden Drehteil ausgebildet sind und mit Lufteinlässen der Mantelwand eines Mundstückes in Strömungsverbindung stehen. Die entsprechenden Lufteinlässe sind mantelwandseitig des Inhalators so platziert, dass sie weder von den Lippen des Benutzers zugehalten werden können, noch durch die Greifhand, die den stabförmigen Körper des Inhalators umfasst. Eine Minimierung der Gefahr eines Zuhaltens ist überdies durch die Ausbildung mehrerer, voneinander beabstandeter Lufteinlässe gegeben. Im Sinne einer guten Verteilung der pulverförmigen Substanz an den Saugluftstrom ist weiter so vorgegangen, dass die Luftdurchlässe axial versetzt zu den dem Mundstück näherliegenden Lufteinlässen angeordnet sind. Das ergibt einen zunächst gegenläufigen Strömungsweg. Zudem erweist es sich als vorteilhaft, dass das Drehteil mit seinem Topfboden die Decke der Vorratskammer bildet, dessen Zentrum eine Führungsöffnung für den als Tauch-Schieber fungierenden Dorn aufweist. Der Topfboden erhält so eine Doppelfunktion: Mittel- oder unmittelbarer Deckel und Führungsloch für den Dorn. Ferner besteht ein vorteilhaftes Merkmal darin, dass der in Tauchrichtung endseitig schraubendreherklingenartig angespitzte Dorn über radiale Flügel mit dem Drehteil drehverbunden ist. Zum einen ist durch die so erzielte schneidenartige Klinge neben einem wirksamen Dreh-Auflockerungseffekt im Zentrumsbereich zugleich das Eintauchen des Dornes in den Pulversee begünstigt und zum anderen besteht eine willkommene Abstützung des Dornes gegenüber dem Drehteil, und was weiter die Ausrichtung der Luftdurchlässe auf die Dosierkammer beibehaltbar macht. Die notwendige lineare Relativbewegung von Dorn und Drehteil zueinander wird mit einfachen Mitteln dadurch erreicht, dass die Topfwand des topfförmigen Drehteils axiale Führungsschlitze aufweist, in denen sich die Flügel führen. Diese Lösung ist weiter gekennzeichnet durch einen vom Mundstück gestellten Zugbegrenzungsanschlag des Dornes, definierend die Entleerungsbereitschaftsstellung der Dosierkammer, die mit ihrem Basiswandabschnitt die Übergabestelle stellt. Die Verschlusskappen-abhängige Lagerung des Dornes ist weiter gekennzeichnet durch eine mundstückseitig liegende, bei Überlast ausklinkende Andockstelle zwischen Dorn und Verschlusskappe. Beim Wiederschließen des Inhalators ergibt sich im Gegenzug das erneute Andocken zwischen Dorn und Verschlusskappe. Eine Ausgestaltung von sogar eigenständiger Bedeutung verkörpert sich sodann darin, dass das Drehteil einen Rotor aufweist, dem ein Stator zugeordnet ist, mit bei Rückdrehen des Drehteils in die Dosierkammer eintragend wirkendem Schaufeleffekt. Hierüber ist das Nachbringen und auch die Dichte der Pulvermenge in der Dosierkammer gleich haltbar. Hinzu kommt ein im Umfeld gegebener Auflockerungseffekt, der das Stocken von Pulverpartien ausschließt. Rückdrehen meint Abschrauben der Verschlusskappe und die damit zusammenhängende Ladewirkung der bzw. auf die Dosierkammer. Im Einzelnen ist das Schaufelwerk durch von einer Ringscheibe des Bodens des Drehteils ausgehenden, Steg getragenen Rotorblättern gebildet. Letztere weisen lanzetten- oder sichelförmigen Umriss auf. Es sind zwei diametral einander gegenüberliegende Rotorblätter realisiert. Baulich ist konkret weiter so vorgegangen, dass die Rotorblätter sich im Wesentlichen auf einem Viertelsektor erstrecken mit einer radial auf das Zentrum des Dornes ausgerichteten Flanke und einer etwa rechtwinklig dazu liegenden Schaufelflanke in spaltbelassend tangierender Ausrichtung zum Dorn. Das schließt Quetschwirkstellen aus. Die bspw. an einem Träger anhaftende medizinische Substanz wird nicht von diesem abgerieben. Sodann ist vorgesehen, dass die Flanken in einer gemeinsamen Diametralen liegen. Auch die weitere bauliche Ausprägung ist schaufelwirksam, jedoch medikamentenschonend ausgebildet, indem der Rotor den Stator so untergreift, dass der Stator als von der Innenwand der Vorratskammer radial einwärts abragender, frei in eine Umlaufbahn des Rotors reichender Vorsprung ausgebildet ist. Der Stator weist trapezförmigen Umriss auf und wurzelt mit seiner Basis in der Innenwand der Vorratskammer. Die Umlaufbahn ist axial durch die Unterseite der Ringscheibe des Drehteils und die ihr zugewandte Innenseite der Rotorblätter begrenzt. Weiter besteht eine zuordnungsgünstige Ausgestaltung darin, dass der Stator umrissmäßig unter dem einen Zwischenraum zwischen zwei Rotorblättern belassenden Viertelsektor liegt. Das erbringt eine ausreichend große Montageöffnung. Sowohl dichtungs- als auch führungsmäßig vorteilhaft ist es, wenn die Führungsöffnung im Drehteil durch eine den zylindrischen Abschnitt des Dornes umfassende Dichtbuchse ausgebildet ist. Es kann sich um Gummi oder gummiähnliches Material handeln. Sich auf dem Schaft des Dornes absetzende pulverförmige Substanz wird durch die Dichtbuchse abgestreift. Es kommt nicht zu einer Verfälschung der ausgabebereiten Dosis. Eine gleichfalls dichtungsbezogene Maßnahme der Spendermechanik ergibt sich durch einen zwischen Innenwand der Vorratskammer und dem Drehteil mit Vorspannung eingesetzten Dichtring. Auch hier kann Gummi oder gummiartiges Material Verwendung finden. Sodann ist vorgesehen, dass der Dichtring in Ringnuten beider Teile passend eingeschnäppert ist, wobei die am Drehteil befindliche Ringnut als V-förmige Kerbnut realisiert und die höhengleich dazu liegende Ringnut der Vorratskammer halbrund ausgebildet ist. Erstere fungiert mit als Drehführung des Drehteils. Endlich wird vorgeschlagen, dass die Verschlusskappe als Schraubkappe ausgebildet ist und mit dem Mundstück über Drehmitnahmemittel zusammenwirkt. Letztere sind klauenkupplungsartig und treten bei Gewindetrennung außer Eingriff.

Der Gegenstand der Erfindung ist nachstehend anhand eines zeichnerisch veranschaulichten Ausführungsbeispieles näher erläutert. Es zeigt:
- Fig. 1: den erfindungsgemäßen Inhalator im Vertikalschnitt, vergrößert, in kappenverschlossener Grundstellung,
- Fig. 2: die Draufsicht hierzu,
- Fig. 3: den Inhalator in Seitenansicht,
- Fig. 4: den Inhalator im Schnitt wie Fig. 1, jedoch bei abgenommener Verschlusskappe und so die Entnahme-Bereitschaftsstellung verkörpernd,
- Fig. 5: eine Herausvergrößerung der Fig. 1 mit in einer Zwischenstellung befindlichem Dorn, wobei sich die Dosierkammer auf Höhe des Stators erstreckt,
- Fig. 6: den Schnitt gemäß Linie VI-VI in Fig. 5,
- Fig. 7: eine Detaildarstellung des Drehteils mit Rotor und Stator in Froschperspektive, zeigend die schneidenartige Gestalt des unteren Dornendes,

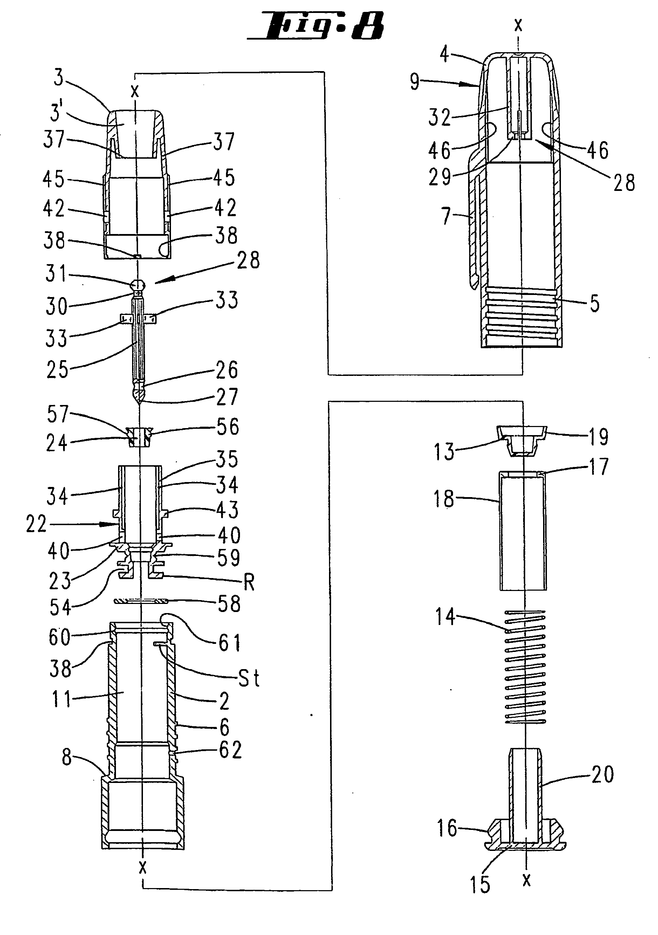

- Fig. 8: eine Explosionszeichnung der den Inhalator bildenden Teile, und zwar bezüglich aller Teile im Vertikalschnitt, hinsichtlich des Dornes im Teilschnitt.

Der in der Zeichnung dargestellte Inhalator 1 ist als bequem mitführbares, kurzstabförmiges Taschengerät realisiert. Formbestimmend ist dabei ein abgesetztes, zylindrisches Gehäuse 2.

Das zylindrische, röhrchenartige Gehäuse 2 geht kopfseitig des Inhalators 1 in ein aufgesetztes Mundstück 3 über. Das ist mundgerecht abgeflacht und lässt sich mittels einer becherförmigen Verschlusskappe 4 schützend überfangen.

Die Verschlusskappe 4 ist als Schraubkappe realisiert. Ein ihr zugeordnetes Innengewinde 5 greift in korrespondierendes Außengewinde 6 an der Mantelwand des Gehäuses 2 ein. Im Ansatzbereich des Mundstückes 3 ist der Verschlusskappe 4 ein Clip 7 angeformt.

Fußseitig tritt der Stirnrand der becherförmigen Verschlusskappe 4 anschlagbegrenzend und abdichtend gegen eine Ringschulter 8, erzielt aufgrund des oben genannten Absatzes des zylindrischen Gehäuses 2.

Den axialen Schraubhub des Gewindeeingriffs 5/6 nutzend, fungiert die Verschlusskappe 4 zugleich als Betätigungshandhabe 9 zur Ausbringung einer pulverförmigen Substanz 10 in reproduzierbaren Teilmengen 10', welche Substanz in einer Vorratskammer 11 des Gehäuses 2 gegebenenfalls nachfüllbar aufgenommen ist. Die jeweils eine Teilmenge 10' an eine außerhalb der Vorratskammer 11 liegende Übergangsstelle Ü fördernde Dosiervorrichtung ist in ihrer Ganzheit mit D bezeichnet.

Bezüglich des dosierfähigen Gutes handelt es sich um eine medizinische pulverförmige Substanz 10, bspw. von der Eigenschaft, dass saugstromtransportfähige Grundkörper (Laktose) als Vehikel oberflächenseitig die daran anhaftenden, mikronisierten Arzneimittel-Feinpartikel tragen.

Der Dosiervorrichtung D nachgeschaltet ist ein sogenannter Dispergierbereich, in welchem durch den Benutzer ein Saugluftstrom S erzeugt wird, der die exakt abgeteilte Teilmenge 10' der Substanz 10 in der Übergabestelle Ü restfrei abträgt. Der zum Mundstück 3 leitende Saugluftkanal trägt das Bezugszeichen 12.

Den unteren Abschluss der Vorratskammer 11 bildet ein topfförmiger Druckboden 13. Der steht in Richtung des Mundstückes 3 unter Federbelastung. Die entsprechende Druckfeder trägt das Bezugszeichen 14. Die stützt sich mit der fußseitigen Endwindung an einer das Gehäuse 2 dort schließenden Bodenkappe 15 ab. Letztere steht in Rasteingriff zum dort querschnittsgrößeren Abschnitt des Gehäuses 2. Der entsprechende Rastkragen 16 greift in eine passende Ringnut des Gehäuses 2 ein.

Die kopfseitige Endwindung der vorgespannten Druckfeder 14 belastet eine Innenschulter 17 eines Hohlkolbens 18 der kolbenförmigen Einrichtung 13/18. Der gestuft topfförmige Druckboden 13 ist mit der Innenschulter 17 rastverbunden.

Der Topfrand des Druckbodens 13 stellt eine Ringlippe 19, die aufgrund ihres gummielastischen Materiales die Wandung der Vorratskammer 21 verlustfrei abstreift.

Sodann geht von der Bodenkappe 15 zentral gelegen ein Stehzapfen 20 aus. Der ist hohl und bildet zusammen mit dem Hohlkolben 18 eine Federkammer 21 für die Druckfeder 14.

Mundstückseitig schließt die Vorratskammer 11 mit einem topfförmigen Drehteil 22 ab. Das bildet mit seinem Topfboden die das Gehäuse 2 überfangende Decke 23 der Vorratskammer 11.

Im Zentrum der Decke 23 ist eine Führungsöffnung 24 belassen. Diese mittel- oder unmittelbar ausgeführte Führungsöffnung 24 nimmt als Herzstück der Dosiervorrichtung D einen Dorn 25 auf. Der fungiert zufolge entsprechender Ausstattung als eine linear bewegte Dosierkammer 26 für die auszuhebende Teilmenge 10',stellend einen Tauchschieber. Er bewegt sich in der Längsmittelachse x-x des im Wesentlichen rotationssymmetrisch gestalteten Inhalators 1.

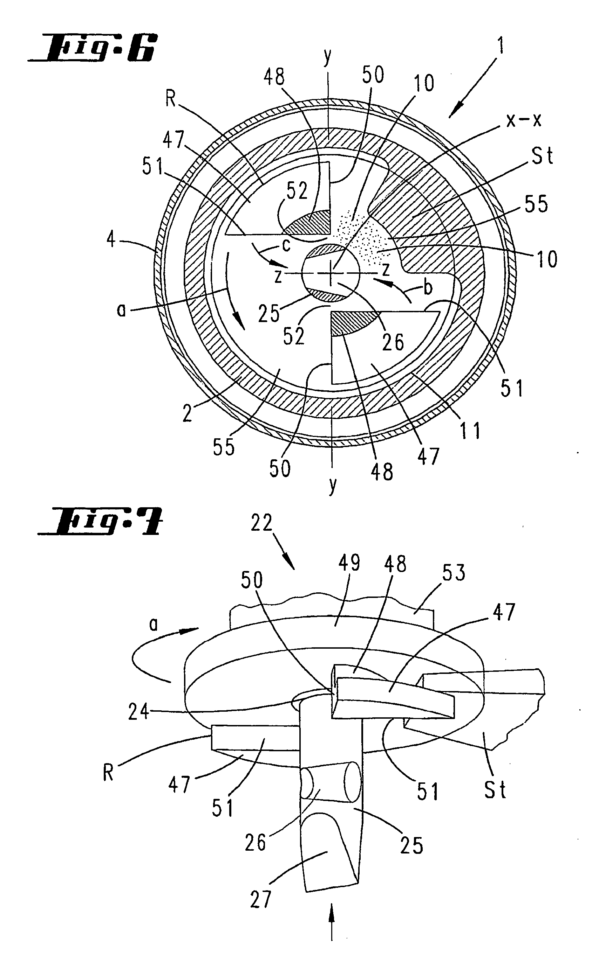

Der Dorn 25 bildet an seinem dem Mundstück 3 abgewandten Ende eine schraubendreherklingenartige Zuspitzung aus. Das hat aufgrund der Drehmitnahme des Dornes 25 im Zentrumsbereich auflockernde Wirkung in Bezug auf den See aus pulverförmiger Substanz 10. Die praktisch spitzdachartige Klinge 27 zeigt zwei spiegelsymmetrische Schrägflanken und schließt basisseitig an einen zylindrischen Schaft des Dornes 25 an. Die Schrägflanken schließen einen Winkel von ca. 60° ein. Der zylindrische Grundquerschnitt des Dornes 25 ist im Bereich der Klinge 27 beibehalten (siehe Fig. 7). Der Hubweg der linear bewegten Dosierkammer 26 berücksichtigt in beiden Endstellungen des Dornes 25 ein Zuhalten des Querschnitts der Führungsöffnung 24 mit dosierkammerfüllender Rakel- bzw. Abstreichwirkung über die Länge der besagten Öffnung 24.

Das mundstückseitige Ende der Verschlusskappe 4 bildet eine bei Überlast ausklinkende Andockstelle 28 zwischen Dorn 25 und Verschlusskappe 4. Das verschlusskappenseitige Rastmittel ist dabei ein ausfederfähiger Hakenkranz. Einwärts gerichtete Nasen 29 der federnden Zungen des Hakenkranzes greifen in eine wespentaillenartige Ringnut 30 des Dornes 25 ein. Nach außen gerichtet setzt sich die Ringnut 30 in einen Rastkopf 31 fort. Der ist in beiden Richtungen durch die Nasen 29 überwindbar. Die rastkopfbildende Materialanhäufung ist etwa linsenförmig.

Die Nasen 29, respektive ihre Federzungen, sind an einem in die Mundstücköffnung 3' ragenden Röhrchen 32 realisiert, welches von der Deckeninnenseite der Verschlusskappe 4 ausgeht. Es wurzelt darin.

Der Dorn 25 ist über speichenartig ausgebildete, radiale Flügel 33 mit dem Drehteil 22 drehverbunden. Die Flügel 33 greifen mit ihren freien Endabschnitten, den Saugluftkanal 12 querend, in axiale Führungsschlitze 34 -es genügen bereits drei- des Drehteiles 22 ein. Die winkelgleich verteilten Führungsschlitze 34 befinden sich innenseitig der Topfwand 35 des topfförmigen Drehteils 22. Die axialen Führungsschlitze 34 sind überdies von solcher Länge, dass der pulverschöpfende Tauchhub des Dornes 25 aus einer Befüllungsebene in der Vorratskammer 11 bis in die geschilderte Übergabestelle Ü oberhalb der Decke 23 gewährleistet ist.

Die definierte Entleerungsbereitschaftsstellung der Dosierkammer 26 ergibt sich durch einen vom Mundstück 3 gestellten Zugbegrenzungsanschlag des Dornes 25. Der ist das Stirnende einer zurückgestülpten Wand des Mundstücks 3, welches so den Ausgang der Führungsschlitze 34 zuhält.

Das Mundstück 3 greift über eine Mantelwand 37 verankernd am Hals des Gehäuses 2 an. Dort ist eine Raststelle 38 zwischen beiden Teilen 2, 3 ausgebildet. Es kann sich um eine irreversible Raststelle 38 handeln. Überdies ist, wie erkennbar, die Decke 23 des Drehteiles 22 durch eine Ringschulter 39 abgestützt überfangen.

Die Dosierkammer 26 ist als im Wesentlichen senkrecht zur Längsmittelachse x-x verlaufende Querbohrung realisiert. In die Entleerungsbereitschaftsstellung überführt, steht die Dosierkammer 26 im Wirkungsbereich des zentralen Saugluftstromes S. Der Dosierkammer 26 ist ein an den Saugluftkanal 12 anschließender Luftdurchlass 40 zugeordnet. Der ist in der Topfwandung 35 des Drehteiles 22 ausgebildet. Es handelt sich um radiale Bohrungen. Sie erstrecken sich in Bodennähe des topfförmigen Drehteils 22, also auf Höhe oder kurz oberhalb der Oberseite der Decke 23.

Erkennbar ist beiden offenen Enden der Dosierkammer 26 ein solcher Luftdurchlass 40 mit radialem Abstand vorgelagert. In diesem Zusammenhang besteht eine Vorkehrung dahingehend, dass dem Ende größeren lichten Durchmessers der von einer konischen Querbohrung gebildeten Dosierkammer 26 ein Luftdurchlass 40 kleineren Durchmessers als dieser zugeordnet ist und dem Ende kleineren lichten Durchmessers der Dosierkammer 26 ein Luftdurchlass 40 größeren Durchmessers als dieser. Hierdurch ergibt sich hinter dem Luftdurchlass 40 kleineren Durchmessers ein größerer Unterdruck mit vorrangiger Austragswirkung bezüglich der dargebotenen Teilmenge 10'. Gleichwohl findet der Austrag, d. h. Entleeren der Dosierkammer 26 von beiden Enden her statt.

Die am topfförmigen, den Dorn 25 abgedichtet führenden Drehteil 22 ausgebildeten Durchlässe 40 sind über einen rückwärtigen Ringraum 41 radial beabstandet noch mit Lufteinlässen 42 strömungsverbunden. Auch die sind als Bohrungen ausgeführt und stellen den Anschluss an die Atmosphäre. Der besagte Ringraum 41 befindet sich zwischen der Außenseite der Topfwand 35 des topfförmigen Drehteils 22 und der Innenseite der Mantelwand 37 des Mundstücks 3.

Erkennbar sind die Luftdurchlässe 40 axial versetzt zu den Lufteinlässen 42 angeordnet. Die Lufteinlässe 42 liegen dem Mundstück 3 näher. Die beschriebene räumliche Abstandslage führt zu einer zunächst gegenläufigen Einströmung an eingesaugter Luft mit Anschluss an den Haupt-Saugluftstrom S. Dies und die Tatsache, dass eine in Erstreckungsrichtung der Dosierkammer 26 liegende Komponente des Saugluftstromes S aufgebaut wird, führt dazu, dass die Dosierkammer 26 restfrei entleert wird. Der Benutzer inhaliert jeweils eine präzise Dosis. Die Übergabestelle Ü wird hier von dem Basisabschnitt der Dosierkammer 26 gestellt.

Förderlich für das entsprechende Entleeren ist die hier entwickelte spezielle Art der Bereithaltung der pulverförmigen Substanz 10 im Schöpfbereich: Hier sind nämlich Bedingungen geschaffen, die das erstrebte strukturgleiche bzw. homogene "Stopfen" der Dosierkammer 26 sicherstellen, gespeist aus einem durchgelockerten Umfeld. Hierzu ist vor allem das Drehteil 22 in weiterbildender Weise herangezogen. Es weist einen im oberen Bereich der Vorratskammer 11 agierenden Rotor R auf. Dem ist ein Stator St zugeordnet. Unter Nutzung der Rotation des Drehteiles 22 ergibt sich neben einem Auflockern zugleich ein Pulver in die Dosierkammer 26 eintragend wirkender Schaufeleffekt beim Rückdrehen des Drehteils 22, d. h. beim Abschrauben der Verschlusskappe 4 unter Nutzung derselben als Betätigungshandhabe 9. Das entsprechende Mitschleppen liegt auch bezüglich des über die Flügel 33 radial drehgesicherten Dornes 25 vor, so dass es kein Verstellen der Achse der Dosierkammer 26 zu den Luftdurchlässen 40 kommt. Selbst die Mantelwand 37 könnte in die Drehfixierung einbezogen sein durch formschlüssige Verbindungsmittel. Im allgemeinen reicht sogar schon eine reibungsschlüssige Mitnahme, bspw. über den den Ringraum 41 zum mundstückseitigen Ende hin zuhaltenden Ringbund 43. Der geht von der Mantelwand der Topfwand 35 aus und liegt mit seiner Randkante an der Innenseite der Mantelwand 37 des Mundstücks 3 an.

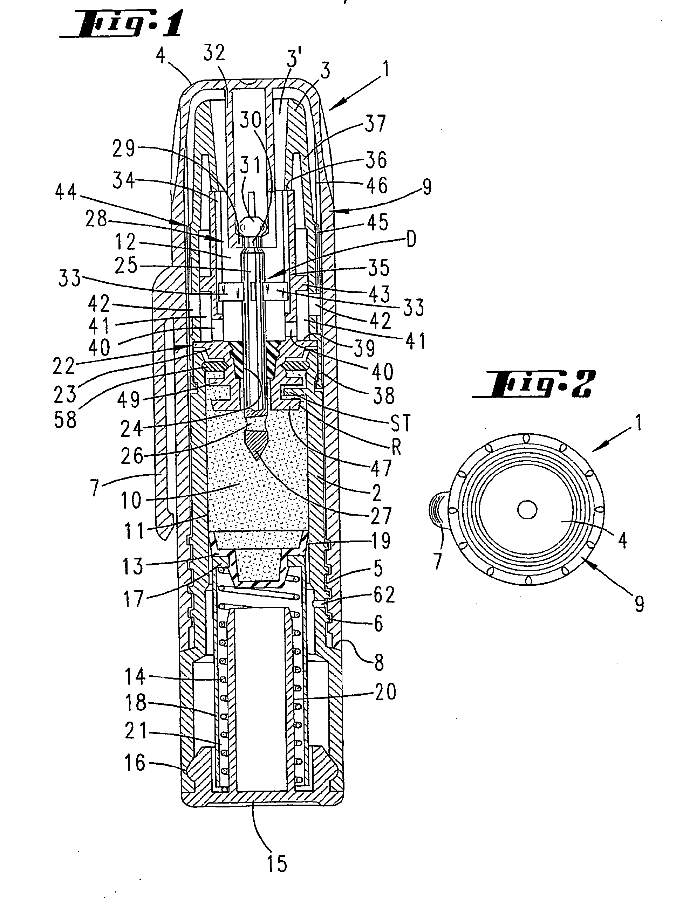

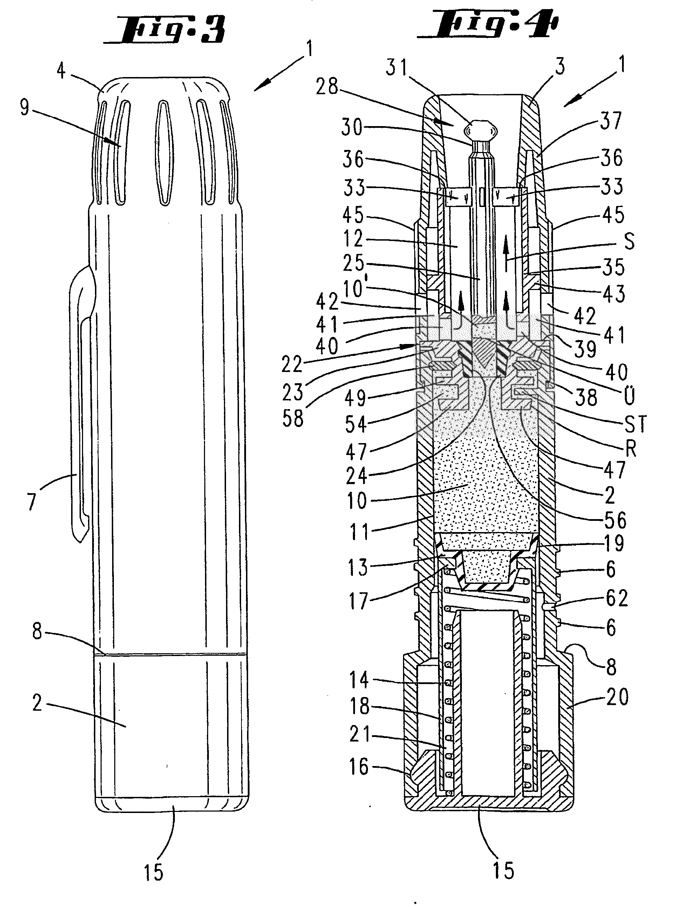

Wie den Fig. 1 und 4 entnehmbar, geschieht die Drehmitnahme zwischen Mundstück 3 und der sich schraubabhebenden Verschlusskappe 4 durch eine Klauenkupplung 44 zwischen beiden. Die besteht aus einer Längszahnung 45 an der Mantelwand 37 des Mundstücks 3, welche Längszahnung in korrespondierende Zahnlücken 46 an der Innenseite der Verschlusskappe 43 eingreifen. Schaufelbildend sind zwei Rotorblätter 47. Die weisen im Grunde sichelförmigen Umriss auf. Die beiden Rotorblätter 47 befinden sich, bezogen auf die Längsmittelachse x-x des Inhalators 1 in diametraler Gegenüberlage. Sie sind an zentrumsbeabstandeten, axial verlaufenden Stegen 48 gehaltert. Die wurzeln in der Unterseite eines Armes oder einer Ringscheibe 49 des den Rotor R stellenden Drehteils 22.

Die vom Boden bzw. der Decke 23 des Drehteils 22 vorratskammerseitig abragenden, freistehenden Rotorblätter 47 sind in einer solchen diametralen Gegenüberlage positioniert, dass sie in Umlaufrichtung genügend beabstandet sind. Geometrisch nehmen sie im Wesentlichen einen Viertelsektor des kreisrunden Querschnitts der Vorratskammer 11 ein. Es sei auf Fig. 6 verwiesen. Die beiden Rotorblätter 47 weisen je eine sich radial auf das Zentrum des Dornes 25 ausgerichtete Flanke 50 auf sowie je eine rechtwinklig dazu liegende Schaufelflanke 51. Die verläuft in spaltbelassendem Abstand zur Mantelwand des Dornes 25. Der Spalt trägt das Symbol 52. So ist zerreibende Wirkung vermieden. Erkennbar sind die Flanken 50 diametral. Die gemeinsame Diametrale der Flanken 50 ist in Fig. 6 mit y-y bezeichnet. Die raumparallelen Schaufelflanken 51 erstrecken sich senkrecht zur Diametralen y-y und raumparallel zur Querbohrungsachse z-z der Dosierkammer 26, welche wiederum mit der Bohrungsachse der Luftdurchlässe 40 zusammenfällt.

Die Ringscheibe 49 oder zwei Arme, in denen die Rotorblätter 47 wurzeln, setzt sich über eine Ringwand 53 in die Decke 23 des Drehteils 22 fort.

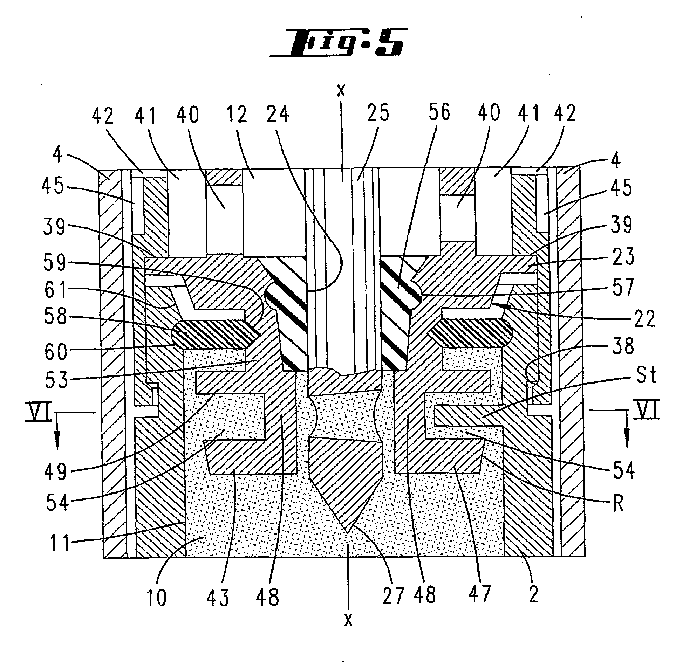

Fig. 5 veranschaulicht besonders deutlich, dass der Rotor R den Stator St so untergreift, dass der Stator St als von der Innenwand der Vorratskammer 11 radial einwärts abragender, frei in eine Umlaufbahn 54 des Rotors R reichender Vorsprung ausgebildet ist. Erkennbar wird die Umlaufbahn 54 axial durch die Unterseite der Ringscheibe 49 des Drehteils 22 und die ihr zugewandte Innenseite der Rotorblätter 47 begrenzt. Der umlaufbahnbildende axiale Abstand ist deutlich größer als die in dieser Richtung gemessene Dicke des Stators St, sprich Vorsprunges, beträgt. So kommt es auch hier nicht zu mechanischen Belastungen gegenüber der reibungsempfindlichen, auszugebenden pulverförmigen Substanz 10.

Der Stator St besitzt trapezförmigen Umriss. Seine kreisbogenförmige Basis wurzelt in der Innenwand der Vorratskammer 11. Die Basis ist dabei so bemessen, dass der sich nach radial innen flächenmäßig verjüngende Stator St umrissmäßig unter dem einen Zwischenraum 55 zwischen zwei Rotorblättern 47 belassenden Viertelsektor liegt. Das stellt, wie aus Fig. 6 entnehmbar, zugleich eine ausreichende Montageöffnung für das Einklinken des Stators in die Umlaufbahn 54.

Der radiale Vorsprung des Stators St nach innen ist von solcher radialer Länge, dass das Plateau des Trapezes ebenfalls spaltbildend vor der Außenseite des Steges 48 endet.

Der Schaufeleffekt wird aus Fig. 6 unter Beachtung der Pfeile deutlich. Pfeil a gibt die Rückdrehrichtung des Drehteiles 22 an. Die Schaufelflanken 51 fungieren so als das davor liegende Pulver schiebende Brust. Pfeil b zeigt die annähernde Einschaufelrichtung bezüglich des größeren lichten Durchmesser aufweisenden Endes der Dosierkammer 26. Pfeil c gibt die entsprechende Wirkung am anderen Rotorblatt 47 an, hier also auch bezüglich der schaufelnden Wirkung der Schaufelflanke 51. Der Stator St steht dabei gleichsam als ortsfeste Schikane im Weg der Umlaufbahn 54. Die pulverförmige Substanz 10 wird durch die der einlenkend wirkenden Trapezflanke näherliegende Schaufelflanke 51 rasch kammerfüllend verlagert, so dass es, wie schon ausgeführt, zu stets gleichen Füllbedingungen kommt. Die Dosierkammer 26 bewegt sich in mehreren Drehungen ansteigend durch die Zone der Dosiervorrichtung D, bis sie mit ihrer Übergabestelle Ü die Oberseite der Decke 23 des topfförmigen Drehteils 22 erreicht hat.

Es wird auch kein dem Dornmantel etwa anhaftendes Pulvergut dosisverfälschend mitgeschleppt, dies zufolge der abstreifend wirkenden Führungsöffnung 24. Letztere ist nicht unmittelbar vom Drehteil 22 gebildet, sondern durch eine diese Durchtrittsstelle auskleidende Dichtbuchse 56. Letztere besteht aus gummielastischem Material und ist in die Decke 23 über Rastmittel 57 eingeklipst gehalten. Sie reicht ebenenmäßig oben bis auf Höhe der Oberseite der Ringscheibe 49.

Es gibt aber auch kein radial außenliegendes Schlupfloch für Pulververluste, denn zwischen Drehteil 22 und dem die Vorratskammer 11 bildenden Gehäuse 2 befindet sich gleichfalls ein Dichtelement. Erreicht ist das durch einen zwischen Innenwand der Vorratskammer 11 und dem Drehteil 22 eingesetzten Dichtring 58 aus gummielastischem Material. Besagter Dichtring 58 ist unter Vorspannung eingesetzt. Der Dichtring 58 ist in Ringnuten beider Teile 2, 22 passend eingeschnäppert. Die am Drehteil 22 befindliche Ringnut trägt das Bezugszeichen 59. Sie ist als V-förmige Kerbnut realisiert. Der Öffnungswinkel der im Bereich der Ringwand 53 liegenden Ringnut 59 beträgt ca. 90°. Die Nutkontur hat zentrierende wie drehführende Wirkung. Die andere, höhengleich dazu liegende Ringnut 60 befindet sich an der Innenseite des Gehäuses 2, und zwar im oberen Eingangsbereich der Vorratskammer 11. Hier liegt bezüglich des Querschnitts der umlaufenden Ringnut 60 eine halbrunde Gestalt vor. Montageerleichternd ist eine der Ringnut 60 vorgeschaltete, rotationssymmetrische Auflaufschräge 61.

Der als Hebedorn gestaltete Dorn 25 kann bezüglich des Volumens seiner Dosierkammer 26 variiert werden, d. h. das Herzstück der Dosiervorrichtung D braucht lediglich ausgetauscht zu werden, um eine andere, genau reproduzierbare Dosierung von Teilmengen 10' zu erzielen.

Der kolbenartig wirkende Druckboden 13 wird in seiner Beweglichkeit gegenüber dem Zylinderraum, gestellt vom mittleren Abschnitt des Gehäuses 2, nicht beeinträchtigt, da das Gehäuse dort eine im Rücken der Ringlippe 19 liegende Luftausgleichsöffnung 62 besitzt.

Der topfförmige Druckboden 13 weist eine zentrale, der Vorratskammer 11 abgewandte Einziehung auf. Die ist innen von solcher Tiefe, dass der in Grundstellung die Rotorblätter 47 axial nach unten überragende Endabschnitt des Dornes 25 darin unterkommt.

Im folgenden Text werden Beispiele für Inhalatoren angegeben, wobei die einzelnen Beispiele nummeriert sind, um die Bezugnahme auf Merkmale einzelner Beispiele in anderen Beispielen zu erleichtern. Nicht alle der unten genannten Beispiele sind unmittelbare Ausführungsbeispiele der beanspruchten Erfindung. Merkmale aus den Beispielen können jedoch mit Merkmalen der Ansprüche kombiniert werden und können das Verständnis der Erfindung erleichtern.
1. Inhalator (1) für pulverförmige, insbesondere medizinische Substanzen (10), mit einem zu einem Mundstück (3) führenden Saugluftkanal (12), ferner einer Vorratskammer (11) für die Substanz (10) und einer linear bewegten Dosierkammer (26) zum Abteilen einer bestimmten Substanzmenge aus der Vorratskammer (11) in den Bereich einer Übergabestelle (Ü) an den Saugluftstrom (S), dadurch gekennzeichnet, dass eine in Erstreckungsrichtung der Dosierkammer (26) liegende Komponente des Saugluftstromes (S) die Dosierkammer (26) entleert.
2. Inhalator nach Beispiel 1 oder insbesondere danach, dadurch gekennzeichnet, dass die Dosierkammer (26) als Querbohrung eines Verschlusskappen-abhängig verlagerbaren Dornes (25) gestaltet ist.
3. Inhalator nach einem oder mehreren der vorhergehenden Beispiele oder insbesondere danach, gekennzeichnet durch eine konische Querbohrung.
4. Inhalator nach einem oder mehreren der vorhergehenden Beispiele oder insbesondere danach, dadurch gekennzeichnet, dass der Dosierkammer (26) ein an den Saugluftstrom (S)anschließender Luftdurchlass (40) zugeordnet ist.
5. Inhalator nach einem oder mehreren der vorhergehenden Beispiele oder insbesondere danach, dadurch gekennzeichnet, dass beiden offenen Enden der Dosierkammer (26) je ein Luftdurchlass (40) vorgelagert ist.
6. Inhalator nach einem oder mehreren der vorhergehenden Beispiele oder insbesondere danach, dadurch gekennzeichnet, dass dem Ende größeren lichten Durchmessers der Dosierkammer (26) ein Luftdurchlass (40) kleineren Durchmessers als dieser zugeordnet ist und dem Ende des kleineren lichten Durchmessers ein Luftdurchlass (40) größeren Durchmessers als dieser.
7. Inhalator nach einem oder mehreren der vorhergehenden Beispiele oder insbesondere danach, dadurch gekennzeichnet, dass die Luftdurchlässe (40) an einem topfförmigen, den Dorn (25) führenden Drehteil (22) ausgebildet sind und mit Lufteinlässen (42) der Mantelwand (37) des Mundstückes (3) in Strömungsverbindung stehen.
8. Inhalator nach einem oder mehreren der vorhergehenden Beispiele oder insbesondere danach, dadurch gekennzeichnet, dass die Luftdurchlässe (40) axial versetzt zu den dem Mundstück (3) näherliegenden Lufteinlässen (42) angeordnet sind.
9. Inhalator nach einem oder mehreren der vorhergehenden Beispiele oder insbesondere danach, dadurch gekennzeichnet, dass das Drehteil (22) mit seinem Topfboden die Decke (23) der Vorratskammer (11) bildet, dessen Zentrum eine Führungsöffnung (24) für den als Tauch-Schieber fungierenden Dorn (25) aufweist.
10. Inhalator nach einem oder mehreren der vorhergehenden Beispiele oder insbesondere danach, dadurch gekennzeichnet, dass der in Tauchrichtung endseitig schraubendreherklingenartig angespitzte Dorn (25) über radiale Flügel (33) mit dem Drehteil (22) drehverbunden ist.
11. Inhalator nach einem oder mehreren der vorhergehenden Beispiele oder insbesondere danach, dadurch gekennzeichnet, dass die Topfwand (35) des topfförmigen Drehteils (22) axiale Führungsschlitze (34) aufweist, in denen sich die Flügel (33) führen.
12. Inhalator nach einem oder mehreren der vorhergehenden Beispiele oder insbesondere danach, gekennzeichnet durch einen vom Mundstück (3) gestellten Zugbegrenzungsanschlag (36) des Dornes (25), definierend die Entleerungsbereitschaftsstellung der Dosierkammer (26), die mit ihrem Basiswandabschnitt die Übergabestelle (Ü) stellt.
13. Inhalator nach einem oder mehreren der vorhergehenden Beispiele oder insbesondere danach, gekennzeichnet durch eine mundstückseitig liegende, bei Überlast ausklinkende Andockstelle (28) zwischen Dorn (25) und Verschlusskappe (4).
14. Inhalator nach einem oder mehreren der vorhergehenden Beispiele oder insbesondere danach, dadurch gekennzeichnet, dass das Drehteil (22) einen Rotor (R) aufweist, dem ein Stator (St) zugeordnet ist, mit bei Rückdrehen des Drehteils (22) in die Dosierkammer (26) eintragend wirkendem Schaufeleffekt.
15. Inhalator nach einem oder mehreren der vorhergehenden Beispiele oder insbesondere danach, gekennzeichnet durch von einer Ringscheibe (49) des Bodens des Drehteils (22) ausgehende, steggetragene Rotorblätter (47).
16. Inhalator nach einem oder mehreren der vorhergehenden Beispiele oder insbesondere danach, dadurch gekennzeichnet, dass die Rotorblätter (47) sichelförmigen Umriss aufweisen.
17. Inhalator nach einem oder mehreren der vorhergehenden Beispiele oder insbesondere danach, gekennzeichnet durch zwei einander gegenüberliegende Rotorblätter (47).
18. Inhalator nach einem oder mehreren der vorhergehenden Beispiele oder insbesondere danach, dadurch gekennzeichnet, dass die Rotorblätter (47) sich im Wesentlichen auf einem Viertelsektor erstrecken mit einer radial auf das Zentrum des Dornes (25) gerichteten Flanke (50) und einer etwa rechtwinklig dazu liegenden Schaufelflanke (51) in spaltbelassend tangierender Ausrichtung zum Dorn (25).
19. Inhalator nach einem oder mehreren der vorhergehenden Beispiele oder insbesondere danach, dadurch gekennzeichnet, dass die Flanken (50) in einer gemeinsamen Diametralen (y-y) liegen.
20. Inhalator nach einem oder mehreren der vorhergehenden Beispiele oder insbesondere danach, dadurch gekennzeichnet, dass der Rotor (R) den Stator (St) so untergreift, dass der Stator (St) als von der Innenwand der Vorratskammer (11) radial einwärts abragender, frei in eine Umlaufbahn (54) des Rotors (R) reichender Vorsprung ausgebildet ist.
21. Inhalator nach einem oder mehreren der vorhergehenden Beispiele oder insbesondere danach, dadurch gekennzeichnet, dass der Stator (St) trapezförmigen Umriss besitzt mit Basis in der Innenwand der Vorratskammer (11).
22. Inhalator nach einem oder mehreren der vorhergehenden Beispiele oder insbesondere danach, dadurch gekennzeichnet, dass die Umlaufbahn (54) axial durch die Unterseite der Ringscheibe (49 des Drehteils (22) und die ihr zugewandte Innenseite der Rotorblätter (47) begrenzt ist.
23. Inhalator nach einem oder mehreren der vorhergehenden Beispiele oder insbesondere danach, dadurch gekennzeichnet, dass der Stator (St) umrissmäßig unter dem einen Zwischenraum (55) zwischen zwei Rotorblättern (47) belassenden Viertelsektor liegt.
24. Inhalator nach einem oder mehreren der vorhergehenden Beispiele oder insbesondere danach, dadurch gekennzeichnet, dass die Führungsöffnung (24) im Drehteil (22) durch eine den zylindrischen Abschnitt des Dornes (25) umfassende Dichtbuchse (56) ausgekleidet ist.
25. Inhalator nach einem oder mehreren der vorhergehenden Beispiele oder insbesondere danach, gekennzeichnet durch einen zwischen Innenwand der Vorratskammer (11) und dem Drehteil (22) mit Vorspannung eingesetzten Dichtring (58).
26. Inhalator nach einem oder mehreren der vorhergehenden Beispiele oder insbesondere danach, dadurch gekennzeichnet, dass der Dichtring (58) in Ringnuten (59, 60) beider Teile passend eingeschnäppert ist, wobei die am Drehteil (22) befindliche Ringnut (59) als V-förmige Kerbnut realisiert und die höhengleich dazu liegende Ringnut (60) der Vorratskammer (11) halbrund ausgebildet ist.
27. Inhalator nach einem oder mehreren der vorhergehenden Beispiele oder insbesondere danach, dadurch gekennzeichnet, dass die Verschlusskappe (4) als Schraubkappe ausgebildet ist und mit dem Mundstück (3) über Drehmitnahmemittel (45/46) zusammenwirkt.

## Patentansprüche

1. Inhalator (1) für pulverförmige, insbesondere medizinische Substanzen (10), mit einem zu einem Mundstück (3) führenden Saugluftkanal (12), ferner einer Vorratskammer (11) für die Substanz (10) und einer linear bewegten Dosierkammer (26) zum Abteilen einer bestimmten Substanzmenge aus der Vorratskammer (11) in den Bereich einer Übergabestelle (Ü) an den Saugluftstrom (S), wobei eine in Erstreckungsrichtung der Dosierkammer (26) liegende Komponente des Saugluftstromes (S) die Dosierkammer (26) entleert, **dadurch gekennzeichnet, dass** die Dosierkammer (26) als Querbohrung eines abhängig von einer Verschlusskappe (4), die Teil des Inhalators (1) ist, verlagerbaren Dornes (25) gestaltet ist und weiter **gekennzeichnet durch** eine mundstückseitig liegende, bei Überlast ausklinkende Andockstelle zwischen dem Dorn (25) und der Verschlusskappe (4), wobei das mundstückseitige Ende der Verschlusskappe (4) die bei Überlast ausklinkende Andockstelle (28) zwischen Dorn (25) und Verschlusskappe (4) bildet, wobei bei der Andockstelle ein Rastmittel ein ausfederfähiger Hakenkranz ist und einwärts gerichtete Nasen (29) der federnden Zungen des Hakenkranzes in eine wespentaillenartige Ringnut (30) eingreifen.

2. Inhalator nach Anspruch 1,
**gekennzeichnet durch** eine konische Querbohrung.

3. Inhalator nach einem der vorhergehenden Ansprüche,
bei dem die Verschlusskappe (4) als Schraubkappe realisiert ist, wobei ein der Verschlusskappe zugeordnetes Innengewinde (5) in ein korrespondierendes Außengewinde (6) an der Mantelwand eines abgesetzten, zylindrischen Gehäuses (2) des Inhalators (1) eingreift.

4. Inhalator nach einem der Ansprüche 1 bis 3,
bei dem die Verschlusskappe (4) als Schraubkappe ausgebildet ist und mit dem Mundstück (3) über Drehmitnahmemittel (45/46) zusammenwirkt.

## Claims

1. Inhaler (1) for powdery, in particular medical, substances (10), with a suction air channel (12) leading to a mouthpiece (3), also with a storage chamber (11) for the substance (10) and with a linearly movable dosing chamber (26) for apportioning a defined amount of substance from the storage chamber (11) into the area of a transfer point (U) to the suction air stream (S), wherein a component of the suction air stream (S) lying in the direction of extent of the dosing chamber (26) empties the dosing chamber (26), **characterized in that** the dosing chamber (26) is configured as a transverse bore of a spindle (25) that is displaceable in dependence on a closure cap (4) which is part of the inhaler (1), and further **characterized by** a docking point between the spindle (25) and the closure cap (4), which docking point lies on the mouthpiece side and disengages if overloaded, wherein the mouthpiece-side end of the closure cap (4) forms the docking point (28), disengaging if overloaded, between spindle (25) and closure cap (4), wherein, at the docking point, a latching means is a ring of hooks capable of resilient deflection, and inwardly directed lugs (29) of the resilient tongues of the ring of hooks engage in a narrow waist-like annular groove (30).

2. Inhaler according to Claim 1, **characterized by** a conical transverse bore.

3. Inhaler according to one of the preceding claims, in which the closure cap (4) is designed as a screw cap, wherein an internal thread (5) assigned to the closure cap engages in a corresponding external thread (6) on the jacket wall of a stepped, cylindrical housing (2) of the inhaler (1).

4. Inhaler according to one of Claims 1 to 3, in which the closure cap (4) is designed as a screw cap and interacts with the mouthpiece (3) via corotating means (45/46).

## Revendications

1. Inhalateur (1) pour substances poudreuses, en particulier médicales (10), comportant un canal d'aspiration d'air (12) menant à un embout buccal (3), par ailleurs une cellule de réserve (11) pour la substance (10) et une cellule de dosage (26) déplacée linéairement pour évacuer une certaine quantité de substance de la cellule de réserve (11) vers la zone d'un point de transfert (Ü) dans le flux d'air d'aspiration (S), un composant du flux d'air d'aspiration (S) se situant dans la direction d'extension de la cellule de dosage (26) vidant la cellule de dosage (26), **caractérisé en ce que** la cellule de dosage (26) est conçue en tant que perçage transversal d'un mandrin (25), déplaçable en dépendance d'un capuchon de fermeture (4), qui est un élément de l'inhalateur (1) et **caractérisé par** ailleurs par un point d'accostage situé du côté de l'embout buccal, qui se décliquète en cas de surcharge, entre le mandrin (25) et le capuchon de fermeture (4), l'extrémité située du côté de l'embout buccal du capuchon de fermeture (4) formant le point d'accostage (28) qui se décliquète en cas de surcharge, entre le mandrin (25) et le capuchon de fermeture (4), près du point d'accostage, un moyen d'enclenchement étant une couronne à crochets susceptible de se détendre et des tenons (29) dirigés vers l'intérieur des languettes élastiques de la couronne à crochets s'engageant dans une rainure annulaire (30) en taille de guêpe.

2. Inhalateur selon la revendication 1,
**caractérisé par** un perçage transversal conique.

3. Inhalateur selon l'une quelconque des revendications précédentes,
sur lequel le capuchon de fermeture (4) est réalisé en tant que capuchon à visser, un taraudage (5) associé au capuchon de fermeture s'engageant dans un filetage (6) correspondant sur la paroi d'enveloppe d'un boîtier (2) cylindrique décalé de l'inhalateur (1).

4. Inhalateur selon l'une quelconque des revendications 1 à 3,
sur lequel le capuchon de fermeture (4) est conçu en tant que capuchon à visser et interagit avec l'embout buccal (3) par l'intermédiaire de moyens d'entraînement en rotation (45/46).
